# EUROPEAN PATENT APPLICATION

(11) **EP 4 650 447 A1**
(43) Date of publication of application: **19.11.2025**
(21) Application number: 24749659.9
(22) Date of filing: 29.01.2024
(51) Int. Cl.: C12N 15/113, C12N 5/10, C12N 15/90, A61K 39/00, C07K 16/28

(54) **COMPOSITION AND METHOD FOR ALLOGENEIC TRANSPLANTATION**

(30) Priority: 30.01.2023 CN 202310081238
(71) Applicant: Nanjing Bioheng Biotech Co., Ltd, Nanjing, Jiangsu 210061 (CN)
(72) Inventor: ZHU, Liping, Nanjing, Jiangsu 210061 (CN); REN, Jie, Nanjing, Jiangsu 210061 (CN); REN, Jiangtao, Nanjing, Jiangsu 210061 (CN)
(74) Representative: Rimini, Rebecca
(86) International application number: PCT/CN2024/074539
(87) International publication number: WO 2024/160180

(57) **Abstract**

Provided are a composition and method for allogeneic transplantation. Provided is an sgRNA combination targeting HLA class I genes, which can knock out HLA-A and HLA-B with high efficiency, and knock out HLA-C with low efficiency. The sgRNA combination can be used for preparing engineered cells with modified HLA class I genes, and the obtained engineered cells can be further used in the prevention and/or treatment of diseases such as cancer, infection or autoimmune diseases.

## Description

### CROSS-REFERENCE TO RELATED APPLICATION

The present disclosure claims the priority to the Chinese patent application with the application No. CN2023100812380, and entitled "COMPOSITION AND METHOD FOR ALLOGENEIC TRANSPLANTATION", filed with China National Intellectual Patent Administration on January 30, 2023, which is incorporated in the present disclosure by reference in its entirety.

### TECHNICAL FIELD

The present invention pertains to the field of gene editing technology and relates to compositions and methods for allogeneic transplantation. Specifically, the present invention relates to an sgRNA combination for modifying HLA class I genes. More specifically, the present invention also relates to a method for preparing engineered cells using the sgRNA combination, a pharmaceutical composition comprising the engineered cells, and its use in the preparation of a medicament for the prevention and/or treatment of cancer, infection or autoimmune diseases.

### BACKGROUND ART

Adoptive cell therapy (ACT) plays an increasingly important role in the treatment of diseases such as tumors. ACT refers to a therapy that transfers therapeutic cells (such as immune cells) into a subject to improve the function and characteristics of the immune system of the subject. The most commonly used therapeutic cell type is T cells, but other cell types, such as NK cells, B cells, dendritic cells, etc., have also been applied. The therapeutic cells can originate from the subject himself (i.e., autologous therapy) or from another individual of the same species (i.e., allogeneic therapy). Allogeneic therapy offers obvious advantages over autologous therapy, such as lower production costs, shorter waiting time for the subject, and a wider range of applications. However, due to reasons such as immune rejection between the subject and exogenous therapeutic cells, allogeneic therapy has not yet been widely used.

In allogeneic transplantation, there are two basic types of immune rejection reactions: host-versus-graft reaction (HVGR) and graft-versus-host reaction (GVHR). Clinically, HVGR is more prevalent. At present, the ways to reduce or avoid HVGR primarily involve knocking out CD52 and knocking out HLA class I genes. Knocking out CD52 can make exogenous therapeutic cells resistant to alemtuzumab (CD52 antibody), thereby avoiding the killing of introduced exogenous therapeutic cells when using alemtuzumab to eliminate T cells in the patient. However, using alemtuzumab will increase the production cost of a cell therapy product. Although knocking out HLA class I genes can partially solve HVGR in patients, exogenous therapeutic cells with HLA class I genes completely knocked-out become susceptible to attack by the NK cells of the patient, which also affects the therapeutic effect of allogeneic therapy.

Therefore, it is necessary to develop a method for allogeneic transplantation to reduce or avoid HVGR, while reducing the attack of host NK cells on exogenous therapeutic cells and improving the therapeutic effect.

### SUMMARY

The present invention aims to provide an sgRNA combination targeting HLA class I genes, which can knock out HLA-A and HLA-B wit high efficiency, and knock out HLA-C with with low efficiency (i.e., retaining most of HLA-C expression), and finally achieve the effect of partially knocking out HLA class I genes. Following knockout of HLA-A and HLA-B, the engagement of engineered cells with TCR is suppressed, and the occurrence of HVGR can be reduced or avoided when the engineered cells are used for allogeneic transplantation. At the same time, retaining most of the HLA-C expression can reduce the killing of allogeneic infused exogenous therapeutic cells by host NK cells, and enhance the survival time and the anti-tumor effect of exogenous therapeutic cells in host cells.

Unless otherwise defined, all technical and scientific terms used herein have the same meaning as commonly understood by one of ordinary skill in the art to which the present application pertains. In order to more easily understand the present application, certain terms are defined below.

### sgRNA

As an emerging gene editing system, CRISPR-Cas has shown great application prospects in the fields of gene expression regulation, pathogenic gene screening and genetic disease treatment. CRISPR-Cas system mainly contains a Cas nuclease and a CRISPR locus, the CRISPR locus consists of a leader sequence, repeat-spacer sequences and palindromic repeat sequences. The leader sequence is responsible for transcribing the repeat-spacer sequences and palindromic repeat sequences to form pre-crRNA, which can form crRNA after maturation. CrRNA can guide the Cas protein to target nucleotide sequences through nucleic acid complementarity to achieve precise cutting. CRISPR system can be classified into 6 types (type I-VI) according to Cas gene structure and repeat spacer sequence structure, among which CRISPR-Cas9 is the most widely used as the representative of type II.

The term "sgRNA", also known as "single guide RNA", refers to a specific RNA sequence that can target a specific gene, which binds to the Cas nuclease and directs it to target sites to perform gene editing. The sgRNA usually contains crRNA and, if necessary, tracrRNA, wherein the crRNA contains a sequence complementary to the target sequence so as to hybridize with the target sequence and guide the RNP complex (Cas nuclease + sgRNA) to specifically bind to the target sequence. The part within sgRNA responsible for complementary pairing with the target DNA is the spacer sequence it contains.

The present invention provides an sgRNA combination targeting HLA class I genes comprising:
(1) HLA-A sgRNA and HLA-B sgRNA-1;
(2) HLA-A sgRNA and HLA-B sgRNA-2; or
(3) HLA-A sgRNA and HLA-B sgRNA-3;
the HLA-A sgRNA contains a spacer sequence as set forth in SEQ ID NO:1; the HLA-B sgRNA-1 contains a spacer sequence as set forth in SEQ ID NO:2; the HLA-B sgRNA-2 contains a spacer sequence as set forth in SEQ ID NO:3; and the HLA-B sgRNA-3 contains a spacer sequence as set forth in SEQ ID NO:4.

### Nucleic Acid, Vector

The term "nucleic acid" may be DNA or RNA. The present invention provides a nucleic acid combination comprising:
(a) a nucleic acid encoding HLA-A sgRNA and a nucleic acid encoding HLA-B sgRNA-1;
(b) a nucleic acid encoding HLA-A sgRNA and a nucleic acid encoding HLA-B sgRNA-2; or
(c) a nucleic acid encoding HLA-A sgRNA and a nucleic acid encoding HLA-B sgRNA-3;
the HLA-A sgRNA contains a spacer sequence as set forth in SEQ ID NO:1; the HLA-B sgRNA-1 contains a spacer sequence as set forth in SEQ ID NO:2; the HLA-B sgRNA-2 contains a spacer sequence as set forth in SEQ ID NO:3; and the HLA-B sgRNA-3 contains a spacer sequence as set forth in SEQ ID NO:4.

The term "vector" is an intermediary nucleic acid molecule used to transfer genetic material into a cell, and in the cell the nucleic acid molecule can be replicated and/or expressed.

In an embodiment, the vector of the present invention includes but is not limited to plasmid, retrovirus, lentivirus, adenovirus, vaccinia virus, Rous sarcoma virus (RSV), polyoma virus and adeno-associated virus (AAV), phage, phagemid, cosmid or artificial chromosome. In an embodiment, the vector also contains an origin of autonomous replication in the host cell, a selectable marker, a restriction enzyme cleavage site, a promoter, a poly-A tail (polyA), a 3'UTR, a 5'UTR, an enhancer, a terminator, an insulator, an operon, a selectable marker, a reporter gene, a targeting sequence and/or a protein purification tag and other elements. Specifically, the vector is a plasmid, a lentiviral vector, an AAV vector, an adenoviral vector and a retroviral vector.

### Engineered Cell and Construction Method thereof

The present invention provides a construction method for an engineered cell and an engineered cell obtained by the construction method, wherein the construction method comprises introducing a Cas nuclease and the sgRNA combination as described above into a cell.

In an embodiment, the Cas nuclease and the sgRNA combination as described above are precomplexed into an RNP complex, which is then introduced into the cell. In another embodiment, the Cas nuclease and the sgRNA as described above are introduced into the cell separately.

In an embodiment, the sgRNA is present in the form of a RNA, an encoding nucleic acid thereof, or a vector, preferably a RNA or an encoding nucleic acid thereof.

In an embodiment, the Cas nuclease is present in the form of a RNA, an encoding nucleic acid thereof, or a vector, preferably mRNA or its analogues.

In an embodiment, the Cas nuclease is a type II nuclease, preferably a Cas9. The Cas9 in the present invention can be SpCas9, SaCas9, NmeCas9, Nme2Cas9, CjCas9, GeoCas9, BlatCas9, PpCas9, SauriCas9, SlugCas9-HF, SchCas9, Nsp2-SmuCas9, etc., preferably SpCas9 or SaCas9.

In an embodiment, the gene encoding HLA class I molecules in the engineered cell is modified. In an embodiment, the modification results in the deletion of HLA-A and/or HLA-B genes. In an embodiment, the modification results in a reduced expression of HLA-A and/or HLA-B genes. Preferably, the reduced expression is the expression reduced to less than 40%, less than 35%, less than 30%, less than 25%, less th an 20%, less than 15%, less than 10%, less than 5%, less than 2%, less than 1%, less than 0.1%, less than 0.01% or less than 0.001% compared with the wild-type cell (assuming that the expression of the gene in the wild-type cell is 100%). In an embodiment, the modification results in substantial retention of the HLA-C gene expression. Preferably, the post-modification expression of the HLA-C gene is retained to greater than 60%, greater than 65%, greater than 70%, greater than 75%, greater than 80%, greater than 85%, greater than 90%, greater than 95% or greater than 99% compared with the wild-type cell (assuming that the expression of the gene in the wild-type cell is 100%).

In an embodiment, the cell is an immune cell.

The term "immune cell" refers to any cell of the immune system that has one or more effector functions (e.g., cytotoxic cell killing activity, secretion of cytokines, induction of ADCC and/or CDC). For example, the immune cell may be a B cell, a T cell, a macrophage, a dendritic cell, a monocyte, an NK cell, or an NKT cell. The immune cell may be obtained from a variety of sources, for example from a subject (e.g., isolated from peripheral blood monocytes, bone marrow, lymph node tissue, cord blood, thymus tissue, tissue from sites of infection, ascites, pleural effusion, spleen tissue, tumors, etc. of the subject), or from in vitro cultured cell line (e.g., Jurkat, SupT1, NK92, etc.), or differentiated from stem cells. Preferably, the immune cell is a T cell or an NK cell, more preferably a T cell. The T cell also may be concentrated or purified. T cells may be at any developmental stage, including but not limited to: a CD4+CD8+ T cell, a CD4+ T cell (for example, Th1 and Th2 cells), CD8+ T cell (for example, cytotoxic T cell), a CD4-CD8- T cell, a tumor infiltrating cell, a memory T cell, a naive T cell, a γδ-T cell, an αβ-T cell, etc. The T cell described in the present invention can be obtained by a variety of techniques known to those skilled in the art, such as obtaining it by isolating from the blood of a subject through Ficoll.

In another embodiment, the cell is a stem cell.

The term "stem cell" refers to a primitive cell with the potential for self-replication, multi-lineage differentiation and homing. It is the origin cell of an organism, and the ancestral cell that gives rise to various tissues and organs of the human body. During the process of cell differentiation, the uneven distribution of regulatory differentiation proteins in the cytoplasm causes one daughter cell irreversibly proceeds toward terminal differentiation, becoming a differentiated cell with specific function until it completely loses the ability to divide again, eventually leading to cellular senescence and death. To compensate for this deficiency, the organism retains a portion of undifferentiated primitive cells, which preserve the parental characteristics. This portion of the retained undifferentiated primitive cells is called stem cells, and when needed, these stem cells can generate differentiated cells by division along developmental pathways. The stem cell described in the present invention can be an embryonic stem cell, an adult stem cell (e.g., an umbilical cord blood stem cell, a bone marrow stem cell, a hematopoietic stem cell, a mesenchymal stem cell, etc.), and a pluripotent stem cell (e.g., an induced pluripotent stem cell iPSC, etc.).

The expression of the B2M gene of the engineered cell of the present invention is suppressed or silenced. In an embodiment, the expression of at least one of the endogenous HLA class II gene of the engineered cell of the present invention is suppressed or silenced. In an embodiment, the expression of at least one of the endogenous TCR/CD3 gene of the engineered cell of the present invention is suppressed or silenced. In an embodiment, the expression of at least one of the endogenous TCR/CD3 gene and B2M gene of the engineered cell of the present invention is suppressed or silenced. In an embodiment, the expression of the B2M gene and HLA class II gene of the engineered cell of the present invention is suppressed or silenced. In an embodiment, the expression of at least one of the endogenous TCR/CD3 gene, at least one of the endogenous HLA class II gene and B2M gene of the engineered cell of the present invention is suppressed or silenced. Preferably, the HLA class II gene is selected from HLA-DPA, HLA-DQ, HLA-DRA, TAP1, TAP2, LMP2, LMP7, RFX5, RFXAP, RFXANK and CIITA, preferably selected from RFX5, RFXAP, RFXANK and CIITA. Preferably, the TCR/CD3 gene is selected from TRAC, TRBC, CD3γ, CD3δ, CD3ε and CD3ζ.

In an embodiment, the expression of one or more endogenous genes of the engineered cell of the present invention is suppressed or silenced, the endogenous genes selected from the group consisting of: CD52, GR, dCK and immune checkpoint genes, e.g., PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

### Chimeric Receptor

In an embodiment, the engineered cell of the present invention may also express a chimeric receptor capable of recognizing a target antigen. The chimeric receptor of the present invention comprises a chimeric antigen receptor (CAR), a T cell receptor (TCR), a T cell receptor fusion protein (TFP), a T cell antigen coupler (TAC) or an immune mobilization monoclonal T cell receptor (ImmTAC), etc., preferably a chimeric antigen receptor or a T cell receptor, more preferably a chimeric antigen receptor.

The term "T cell receptor" or "TCR" refers to a membrane protein complex that responds to antigen presentation and participates in T cell activation. The stimulation of TCR is triggered by the major histocompatibility complex molecule (MHC) on the antigen presenting cell, which presents antigen peptides to T cells and bind to the TCR complexs to induce a series of intracellular signaling. TCR is composed of six peptide chains that form heterodimers, which are generally divided into αβ type and γδ type. Each peptide chain includes a constant region and a variable region, wherein the variable region is responsible for binding to specific antigen and MHC molecules. The variable region of TCR may contain an antigen binding domain or be operably linked to an antigen binding domain, wherein the definition of the antigen binding domain is as described below.

The term "T cell antigen coupler" or "TAC" includes three functional domains: (1) a tumor targeting domain, including a single chain antibody, a designed ankyrin repeat protein (DARPin), or other targeting groups; (2) an extracellular domain, a CD3-binding single-chain antibody, so that TAC receptors and TCR receptors are close to each other; and (3) a transmembrane domain and an intracellular domain of a CD4 co-receptor, wherein the intracellular domain is linked to protein kinase LCK, to catalyze the phosphorylation of the immunoreceptor tyrosine activation motif (ITAM) of the TCR complex as an initial step in T cell activation.

The term "T cell receptor fusion protein" or "TFP" refers to a recombinant polypeptide derived from components of TCR, usually composed of a TCR subunit and an antigen binding region linked thereto, and expressed on the cell surface. Wherein, the TCR subunit includes at least part of the TCR extracellular domain, the transmembrane domain, and the TCR intracellular signal domain.

The term "immune mobilizing monoclonal T cell receptor" or "ImmTAC" is composed of an engineered T cell receptor (TCR) and an anti-CD3 scFv, wherein: the engineered TCR can specifically recognize and bind to HLA-peptide complex on the surface of tumor cells with significantly improved affinity, and promotes T cell-mediated effector functions through the interaction of the scFv antibody fragment with CD3.

The term "chimeric antigen receptor" or "CAR" refers to an artificially constructed hybrid polypeptide that generally includes an antigen (e.g., a tumor antigen) binding domain (e.g., an antibody or antigen ligand), a transmembrane domain, an optional co-stimulatory domain, and a primary signaling domain, and each domain is linked by a linker. CARs are able to reposition the specificity and reactivity of T cells and other immune cells to a selected target in a non-MHC-restricted manner. In an embodiment, the functional exogenous receptor of the present invention is a chimeric antigen receptor, which contains a tumor antigen binding domain, a transmembrane domain, one or more co-stimulatory domains, and a primary signaling domain. In an embodiment, the chimeric antigen receptor further contains one or more of the following structures: a signal peptide, a hinge region, a suicide gene, a switch structure, etc.

The term "antigen binding domain" refers to any structure or functional variant thereof that can specifically bind to an antigen, including an antibody that bind to a target antigen or an antigen-binding fragment thereof. Those skilled in the art can determine the antigen to be targeted based on the disease to be treated, thereby designing the corresponding chimeric antigen receptor. The target antigen of the present invention is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gpl30, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof. Preferably, the antigen is selected from CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA and GPRC5D, more preferably selected from CD19, Claudin18.2, MSLN, GPRC5D, CD7, BCMA, and any combination thereof.

The term "antibody" includes monoclonal antibodies (including whole antibodies), polyclonal antibodies, multivalent antibodies, multispecific antibodies (for example, bispecific antibodies), and antibody fragments or synthetic polypeptides carrying one or more CDR sequences capable of exhibiting the desired biological activity, etc. Typically, whole antibodies include two heavy chains and two light chains linked together by disulfide bonds, forming a "Y" shaped structure. Each of the heavy chain and light chain includes a variable region and a constant region, and each of the heavy and light chain variable regions includes three CDRs, which are separated by a more conservative framework region (FR). The variable region is responsible for the recognition and binding of the antigen, while the constant region can mediate the binding of the antibody to the host tissue or factor.

The term "antigen binding fragment" includes an antibody fragment, a ligand or a functional fragment thereof (i.e., a fragment with the ability to bind the antigen). An "antibody fragment" is a portion of an intact antibody, and generally, an antibody fragment contain the antigen binding site of the intact antibody and thus retains the ability to bind to an antigen. Examples of the antigen binding fragment in the present invention include, but are not limited to: Fab, Fab', F(ab')2, Fd fragment, Fd', Fv fragment, scFv, disulfide-linked Fv (sdFv), linear antibody, "diabody" with two antigen binding sites, single domain antibody (e.g., heavy chain variable region VH, light chain variable region VL, nanobody, etc. of an antibody). A "ligand" refers to any molecule or atom that can reversibly bind to an antigen molecule and interact with it. A ligand can be a single atom or ion, or a larger, more complex molecule composed of many atoms. A ligand can be a natural ligand, such as an organic or inorganic molecule, or a synthetic ligand.

The term "transmembrane domain" refers to a polypeptide structure that enables expression of a chimeric antigen receptor on the surface of an immune cell (e.g., a lymphocyte, a NK cell, or a NKT cell), and guides a cellular response of the immune cell against the target cell. In an embodiment, the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, and CD154.

The term "primary signaling domain" refers to a portion of a portion that transduces an effector function signal and guides a cell to perform a specified function, which is responsible for the intracellular primary signal transmission after the antigen binding domain binds to the antigen, thereby leading to the activation of immune cells and immune responses. In an embodiment, the primary signaling domain is a signaling domain of a protein selected from the group consisting of: FcRγ, FcRβ, CD3γ, CD3δ, CD3ε, CD3ζ, CD22, CD79a, CD79b, and CD66d.

The term "co-stimulatory domain" can be an intracellular functional signaling domain from a co-stimulatory molecule. A "co-stimulatory molecule" refers to a cognate binding partner that specifically binds to a co-stimulatory ligand on a T cell, thereby mediating a co-stimulatory response (e.g., proliferation) of the T cell. The co-stimulatory domain includes, but is not limited to, a co-stimulatory signaling domain derived from the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD150 (SLAMF1), CD152 (CTLA4), CD223 (LAG3), CD270 (HVEM), CD272 (BTLA), CD273 (PD-L2), CD274 (PD-L1), CD276 (B7-H3), CD278 (ICOS), CD357 (GITR), DAP10, LAT, NKG2C, SLP76, PD1, LIGHT, TRIM, ZAP70, or any combination thereof.

In an embodiment, the chimeric antigen receptor further contains a hinge region located between the antigen binding domain and the transmembrane domain. The term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, e.g., completely or partially from the extracellular region of CD8, CD4 or CD28, or completely or partially from an antibody constant region. Alternatively, the hinge region may be a synthetic sequence corresponding to a naturally existing hinge sequence, or may be a completely synthetic hinge sequence. All the hinge regions known in the art may be used in the present invention, for example, the hinge region of CD8 α chain, FcγRIII α receptor, IgG4 or IgG1.

In an embodiment, the chimeric antigen receptor further includes a signal peptide. The signal peptide allows the nascent protein to be directed to the endoplasmic reticulum and subsequently to the cell surface when it is expressed in a cell, e.g. a T cell. The core of the signal peptide may contain a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. All the signal peptides known in the art can all be used in the present invention, for example, signal peptides derived from B2M, CD8α, IgG1, GM-CSFRα, etc.

### Pharmaceutical composition

The present invention also provides a pharmaceutical composition comprising the engineered cell described in the present invention. In an embodiment, it further comprises one or more pharmaceutically acceptable excipients.

As used herein, the term "pharmaceutically acceptable excipient" refers to a carrier and/or excipient that is pharmacologically and/or physiologically compatible with the subject and the active ingredient (i.e., capable of inducing the desired therapeutic effect without causing any undesirable local or systemic effects), including but not limited to filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. There are a variety of ways of administering the composition in the art, including but not limited to oral, injection, aerosol, parenteral and topical administration.

The composition of the present invention can also be administered in combination with one or more other agents suitable for treating and/or preventing the disease to be treated. Preferred examples of agent suitable for the combination include known anti-cancer medicaments, such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, e.g., ricin, diphtheria toxin, pseudomonas exotoxin A, DNase and RNase; radionuclides, e.g., iodine 131, rhenium 186, indium 111, iridium 90, bismuth 210, bismuth 213, actinides 225 and astatine 213; prodrugs, e.g., antibody-directed enzyme prodrugs; immunostimulatory agents, e.g., platelet factor 4, melanoma growth stimulating protein, etc.; antibodies or fragments thereof, e.g., anti-CD3 antibodies or fragments thereof, complement activators, heterologous protein domains, homologous protein domains, viral/bacterial protein domains and viral/bacterial peptides. In addition, the pharmaceutical composition of the present invention also can be used in combination with one or more other treatment methods, for example, chemotherapy and radiotherapy.

### Use for prevention and/or treatment

The engineered cell and composition of the present invention can be used as a medicament for the prevention and/or treatment of cancer, infection or autoimmune diseases.

In an embodiment, the cancer that can be prevented and/or treated with the engineered cell or composition of the present invention includes non-solid tumors (such as hematological tumors, e.g., leukemia and lymphoma) and solid tumors. Hematological tumors are cancers of the blood or bone marrow, including but not limited to acute leukemias (such as acute lymphoblastic leukemia, acute myeloid leukemia, acute myelogenous leukemia and myeloblastic, promyelocytic, granulocytic-monocytic, monocytic and erythroleukemia), chronic leukemias (such as chronic myeloid (granulocytic) leukemia, chronic myelogenous leukemia and chronic lymphocytic leukemia), polycythemia vera, lymphomas, Hodgkin lymphoma, non-Hodgkin lymphoma (indolent and high-grade forms), multiple myeloma, Waldenstrom's macroglobulinemia, myelodysplastic syndrome, hairy cell leukemia, Burkitt lymphoma, diffuse large cell lymphoma, mantle cell lymphoma, T-lymphoblastic leukemia/lymphoma (T-ALL/LBL), early pro-T lymphoblastic leukemia (ETP-ALL), extranodal NK/T-cell lymphoma, small lymphocytic lymphoma (SLL), and myelodysplasia. Solid tumors are abnormal masses of tissue that usually do not contain cysts or fluid areas, which can be benign or malignant. Different types of solid tumors are named according to the type of cells from which they are derived (such as sarcomas, carcinomas and lymphomas). Examples of solid tumors include, but are not limited to, fibrosarcoma, myxosarcoma, liposarcoma, mesothelioma, pancreatic cancer, ovarian cancer, peritoneal, omental and mesenteric cancer, pharyngeal cancer, prostate cancer, rectal cancer, renal cancer, skin cancer, small intestine cancer, melanoma, kidney cancer, laryngeal cancer, soft tissue cancer, stomach cancer, testicular cancer, colon cancer, esophageal cancer, cervical cancer, alveolar rhabdomyosarcoma, bladder cancer, bone cancer, brain cancer, breast cancer, anal cancer, eye cancer, intrahepatic cholangiocarcinoma, joint cancer, neck cancer, gallbladder cancer, pleural cancer, nasal cancer, middle ear cancer, oral cancer, vulvar cancer, thyroid cancer and ureteral cancer. Preferably, cancers that can be prevented and/or treated with the engineered cell or composition of the present invention include acute lymphocytic leukemia (ALL), acute myeloid leukemia (AML), T-lymphoblastic lymphoma (T-LBL), early pre-T lymphoblastic leukemia (ETP-ALL) and extranodal NK/T cell lymphoma.

In an embodiment, the engineered cell or composition of the present invention may also be used in combination with one or more additional chemotherapeutic agents, biological formulations, medicaments or treatments. In this embodiment, the chemotherapeutic agent, biological preparation, medicament or treatment is selected from radiation therapy, surgery, antibody reagents, small molecules or any combination thereof.

The present invention will be described in detail below in conjunction with the examples and the accompanying drawings. It should be noted that those skilled in the art should understand that the examples of the present invention are only for illustration, and cannot constitute any limitation to the present invention. In the case of no contradiction, the embodiments in the present application and the features in the embodiments can be combined with each other.

### BRIEF DESCRIPTION OF DRAWINGS

Fig. 1: Results of HLA-A gene knockout using a single sgRNA detected by flow cytometry;
Fig. 2: Results of HLA-B gene knockout using a single sgRNA detected by flow cytometry;
Fig. 3: Results of HLA-C gene knockout using a single sgRNA detected by flow cytometry;
Fig. 4: Results of HLA-A/B/C gene knockout using an sgRNA combination detected by flow cytometry.

### EXAMPLES

### Example 1 Design and synthesis of sgRNA

A suitable PAM site was selected on the signal peptide sequence of HLA-A to design the spacer sequence of sgRNA, the spacer sequence targets the signal peptide region of HLA-A, and an HLA-A sgRNA containing the spacer sequence as set forth in SEQ ID NO: 1 was synthesized.

A suitable PAM site was selected on the signal peptide sequence of HLA-B to design 3 spacer sequences of sgRNA. The spacer sequences targeted the signal peptide region of HLA-B, and HLA-B sgRNA-1, HLA-B sgRNA -2, and HLA-B sgRNA-3 containing the spacer sequences as set forth in SEQ ID NO: 2, SEQ ID NO: 3, and SEQ ID NO: 4, respectively, were synthesized.

Since the signal peptide gene cannot express the signal peptide normally after being edited, HLA-A and HLA-B therefore fail to expressed normally on the cell membrane without the guidance of the corresponding signal peptide, and thus cannot function normally, thereby achieving the knockout effect on HLA-A and HLA-B. In addition, the signal peptides of the classical HLA class I molecules HLA-A, HLA-B, and HLA-C are highly homologous, thus the sgRNA targeting the signal peptide of HLA-A or HLA-B may have a knockout effect on either one, two, or all three genes of HLA-A, HLA-B, and HLA-C.

### Example 2 Knocking out HLA class I molecules in T cells using a single sgRNA

The T cells used in the examples of the present invention are primary human T cells isolated from peripheral blood of healthy donors via density gradient centrifugation using Ficoll-Paque PREMIUM (GE Healthcare).

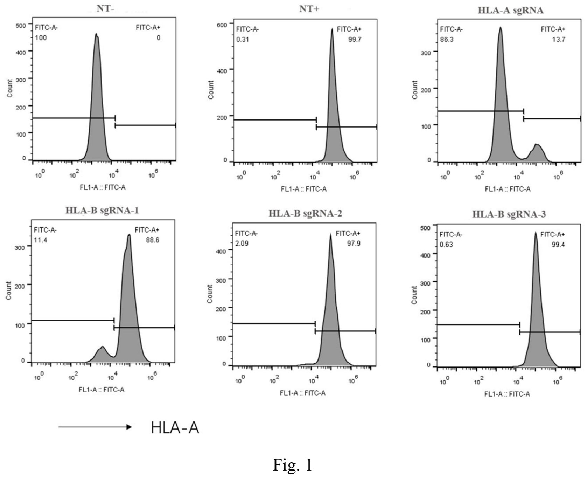

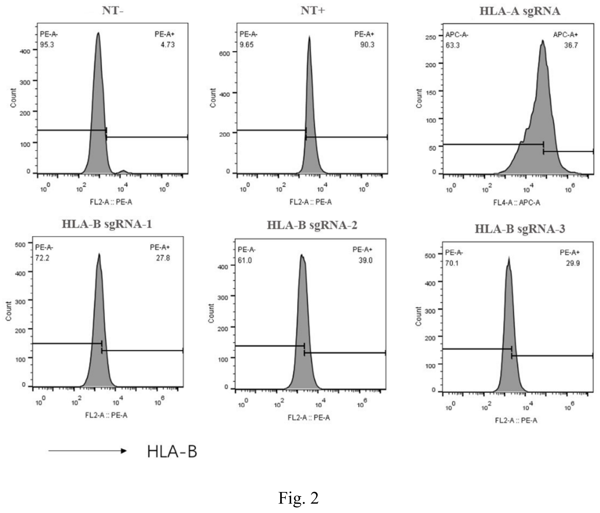

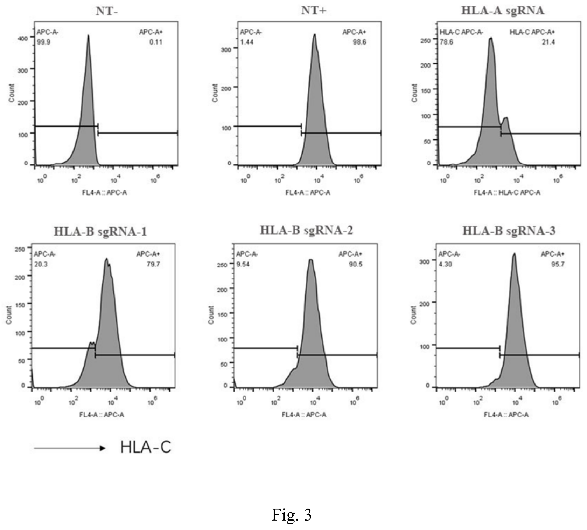

The isolated primary human T cells were cultured with 37°C and 5% CO₂ for 24 hours, then the T cells were stimulated with DynaBeads CD3/CD28 (Thermo Fisher, Cat. No. 40203D) and cultured for 3 days under 37°C and 5% CO₂. Then, 30 µg of Cas9 mRNA was electrotransfected into the activated T cells using a BTX Agile Pulse Max electroporator (Harvard Apparatus BTX) at 500 V and 1.0 ms. After electrotransfection, the T cells were immediately placed in pre-warmed culture medium and cultured at 37°C and 5% CO₂ for 24 hours in the presence of IL-2 (300 IU/mL). On day 4, 10 µg of HLA-A sgRNA, HLA-B sgRNA-1, HLA-B sgRNA-2, HLA-B sgRNA-3, and HLA-B sgRNA-4 were each electrotransfected into the activated T cells using a BTX Agile Pulse Max electroporator (Harvard Apparatus BTX) again at 400 V and 0.7 ms. And in the presence of IL-2 (300 IU/mL), the cells were cultured at 37°C and 5% CO₂ until day 11, and the knockout efficiency of HLA-A, HLA-B, and HLA-C was detected using C6 flow cytometry with BV605 Rat Anti-Human HLA-B (BD Pharmingen, Cat. No. 752616), FITC Mouse Anti-Human HLA-A (BD Pharmingen, Cat. No. 568029), and Alexa Flour 647 anti-human HLA-C Antibody (Biolegend, Cat. No. 568029). T cells not transfected with Cas9 mRNA and sgRNA were served as controls (NT). The control group without HLA-A, HLA-B, or HLA-C antibodies was named "NT-", and the control group with HLA-A, HLA-B, or HLA-C antibodies was named "NT+", establishing the gating basis for flow cytometry detection. The results of knockout efficiency are shown in Table 1 and Figs. 1, 2, and 3.

**Table 1 Knockout efficiency of different sgRNAs on HLA-A, HLA-B and HLA-C genes**

| Numbering | Spacer Sequence | Knockout Efficiency of HLA-A (%) | Knockout Efficiency of HLA-B (%) | Knockout Efficiency of HLA-C (%) |
|---|---|---|---|---|
| HLA-A sgRNA | AGUAGCAGGAGGAGGGUUCG | 86.3 | 63.3 | 78.6 |
| HLA-B sgRNA-1 | AGAGCAGCAGGAGGACGGUU | 11.4 | 72.2 | 20.3 |
| HLA-B sgRNA-2 | GAGCAGCAGGAGGACGGUUC | 2.1 | 61.0 | 9.5 |
| HLA-B sgRNA-3 | AGCAGCAGGAGGACGGUUCG | 0.6 | 70.1 | 4.3 |

As can be seen from Table 1, different sgRNAs have different knockout efficiencies. Among them, HLA-A sgRNA exhibites good knockout efficiency for all three genes HLA-A, HLA-B, and HLA-C, and the knockout efficiency for HLA-A is the highest, reaching 86.3%; the knockout efficiency for HLA-B reaches 63.3%, and the knockout efficiency for HLA-C reaches 78.6%. HLA-B sgRNA-1, HLA-B sgRNA-2, and HLA-B sgRNA-3 only exhibit high knockout efficiencies for the HLA-B gene, and have almost no knockout effect on HLA-A and HLA-C.

From the above results, it can be seen that HLA-A sgRNA can achieve good knockout effects on the three genes HLA-A, HLA-B, and HLA-C. HLA-B sgRNA-1, HLA-B sgRNA-2, and HLA-B sgRNA-3 can achieve efficient knockout effects on the HLA-B gene.

### Example 3 HLA-A sgRNA combined with HLA-B sgRNA-1, HLA-B sgRNA-2, and HLA-B sgRNA-3 to knock out HLA class I molecules in T cells, respectively

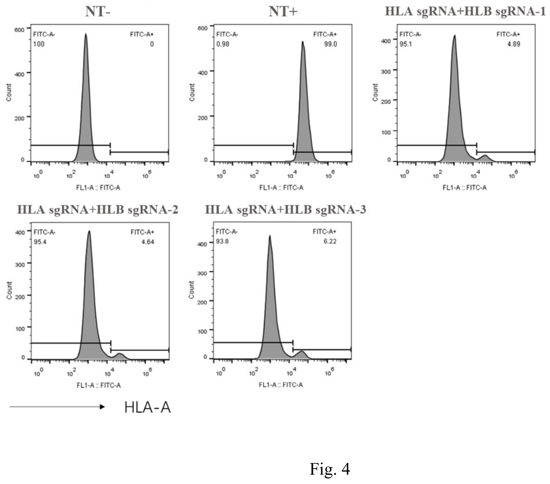

According to the method of Example 1, the 10 µg sgRNA electrotransfected into the activated T cells was replaced with 5 µg HLA-A sgRNA and 5 µg HLA-B sgRNA-1, 5 µg HLA-A sgRNA and 5 µg HLA-B sgRNA-2, 5 µg HLA-A sgRNA and 5 µg HLA-B sgRNA-3, a total of three groups (grouping is shown in Table 2), and the knockout efficiency of HLA-A, HLA-B and HLA-C was detected. T cells untransfected with Cas9 mRNA and sgRNA served as controls. The results of the knockout efficiency are shown in Table 2 and Fig. 4.

**Table 2 Knockout efficiency of sgRNA combination on HLA-A, HLA-B, and HLA-C**

| Grouping | Numbering | Knockout Efficiency of HLA-A (%) | Knockout Efficiency of HLA-B (%) | Knockout Efficiency of HLA-C (%) |
|---|---|---|---|---|
| 1 | HLA-A sgRNA+HLA-B sgRNA-1 | 95.1 | 76.6 | 29.8 |
| 2 | HLA-A sgRNA+HLA-B sgRNA-2 | 95.4 | 73.5 | 33.8 |
| 3 | HLA-A sgRNA+HLA-B sgRNA-3 | 93.8 | 68.0 | 31.6 |

From the knockout results of the combinations of HLA-A sgRNA with HLA-B sgRNA-1, HLA-B sgRNA-2, and HLA-B sgRNA-3 in Table 2, the knockout efficiency on the HLA-A gene was improved after combined knockout, which was higher than that of HLA-A sgRNA alone; the knockout efficiency on the HLA-B gene was slightly improved compared with that of HLA-A sgRNA alone; and the knockout efficiency on the HLA-C gene was about 30%, which was much lower than that of HLA-A sgRNA alone on the HLA-C gene.

Therefore, compared with knockout of HLA-A sgRNA alone, knockout of HLA-A sgRNA in combination with HLA-B sgRNA-1, HLA-B sgRNA-2, and HLA-B sgRNA-3 can improve the knockout efficiencies on the HLA-A and HLA-B genes, and significantly reduce the knockout efficiency on the HLA-C gene.

It should be noted that the above-mentioned are merely for preferred examples of the present invention and not used to limit the present invention. For those skilled in the art, various modifications and changes may be made to the present invention. Those skilled in the art should understand that any amendments, equivalent replacements, improvements, and so on, made within the spirit and principle of the present invention, should be covered within the scope of protection of the present invention.

## Claims

1. An sgRNA combination comprising:
(1) HLA-A sgRNA and HLA-B sgRNA-1;
(2) HLA-A sgRNA and HLA-B sgRNA-2; or
(3) HLA-A sgRNA and HLA-B sgRNA-3;
the HLA-A sgRNA contains a spacer sequence as set forth in SEQ ID NO: 1; the HLA-B sgRNA-1 contains a spacer sequence as set forth in SEQ ID NO:2; the HLA-B sgRNA-2 contains a spacer sequence as set forth in SEQ ID NO:3; and the HLA-B sgRNA-3 contains a spacer sequence as set forth in SEQ ID NO:4.

2. A nucleic acid combination comprising:
(a) a nucleic acid encoding HLA-A sgRNA and a nucleic acid encoding HLA-B sgRNA-1;
(b) a nucleic acid encoding HLA-A sgRNA and a nucleic acid encoding HLA-B sgRNA-2; or
(c) a nucleic acid encoding HLA-A sgRNA and a nucleic acid encoding HLA-B sgRNA-3;
the HLA-A sgRNA contains a spacer sequence as set forth in SEQ ID NO:1; the HLA-B sgRNA-1 contains a spacer sequence as set forth in SEQ ID NO:2; the HLA-B sgRNA-2 contains a spacer sequence as set forth in SEQ ID NO:3; and the HLA-B sgRNA-3 contains a spacer sequence as set forth in SEQ ID NO:4.

3. A vector comprising the nucleic acid combination of claim 2.

4. A method for constructing an engineered cell comprising: introducing the sgRNA combination of claim 1 or the nucleic acid combination of claim 2 or the vector of claim 3, and a Cas nuclease into a cell.

5. The method according to claim 4, **characterized in that** the Cas nuclease is Cas9.

6. An engineered cell obtaining according to the method of claim 4 or 5.

7. The engineered cell according to claim 6, **characterized in that** the cell is an immune cell, and the immune cell is a T cell, a B cell, a macrophage, a dendritic cell, a neutrophil, a monocyte, an NK cell or an NKT cell.

8. The engineered cell according to claim 7, **characterized in that** the cell is a CD4+CD8+ T cell, a CD4+ T cell, a CD8+ T cell, a CD4-CD8- T cell, a tumor infiltrating cell, a memory T cell, a naive T cell, a γδ-T cell or an αβ-T cell.

9. The engineered cell according to claim 6, **characterized in that** the cell is a stem cell, and the stem cell is an embryonic stem cell, an umbilical cord blood stem cell, a bone marrow stem cell, a hematopoietic stem cell, a mesenchymal stem cell or an induced pluripotent stem cell.

10. The engineered cell according to any one of claims 6-9, **characterized in that** the engineered cell further expresses a chimeric receptor capable of recognizing a target antigen, and the chimeric receptor is selected from the group consisting of a chimeric antigen receptor, a T cell receptor, a T cell receptor fusion protein, a T cell antigen coupler, and an immune mobilizing monoclonal T cell receptor antigen.

11. The engineered cell according to claim 10, **characterized in that** the chimeric receptor is a chimeric antigen receptor comprising an antigen binding domain, a transmembrane domain and an intracellular domain, **characterized in that** the intracellular domain is a co-stimulatory domain and/or a primary signaling domain.

12. The engineered cell according to claim 10 or 11, **characterized in that** the target antigen is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof; Preferably, the antigen is selected from the group consisting of CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA, GPRC5D and any combination thereof.

13. The engineered cell according to claim 11, **characterized in that** the transmembrane domain is selected from a transmembrane domain of a protein selected from the group consisting of a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8a, CD9, CD16, CD22, CD33, CD37, CD47, CD64, CD80, CD86, CD94, CD134, CD137, CD154, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CD11d, ITGAE, CD103, ITGAL, CD1 la, LFA-1, ITGAM, CD11b, ITGAX, CD11c, ITGB1, CD29, ITGB2, CD18, LFA-1, ITGB7, TNFR2, DNAM1, SLAMF4, CD84, CD96, CEACAM1, CRT AM, Ly9, CD160, PSGL1, CDIOO, SLAMF6, SLAMF1, SLAMF8, CD162, LTBR, PAG/Cbp, NKp44, NKp30, NKp46, NKG2D and NKG2C.

14. The engineered cell according to claim 11, **characterized in that** the co-stimulatory domain is selected from a intracellular region of a protein selected from the group consisting of LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD1, LIGHT, TRIM, ZAP70 and any combinations thereof.

15. The engineered cell according to claim 11, **characterized in that** the primary signaling domain is selected from a signaling region of a protein selected from the group consisting of FcRγ, FcRβ, CD3γ, CD3δ, CD3ε, CD3ζ, CD5, CD22, CD79a, CD79b, NFAM1, STAM1, STAM2, and Cd66d.

16. The engineered cell according to any one of claims 6-15, **characterized in that** in the engineered cell, expression of one or more genes among endogenous B2M gene, HLA class II gene, and TCR/CD3 gene is suppressed or silenced.

17. The engineered cell according to claim 16, **characterized in that** the endogenous HLA class II gene is selected from the group consisting of HLA-DPA, HLA-DQ, HLA-DRA, TAP1, TAP2, LMP2, LMP7, RFX5, RFXAP, RFXANK, CIITA and any combination thereof; the TCR/CD3 gene is selected from the group consisting of TRAC, TRBC, CD3γ, CD3δ, CD3ε, CD3ζ and any combination thereof.

18. The engineered cell according to any one of claims 6-17, **characterized in that** in the engineered cell, expression of one or more endogenous genes selected from the group consisting of: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3 is suppressed or silenced.

19. A pharmaceutical composition comprising the engineered cell of any one of claims 6-18, and one or more pharmaceutically acceptable excipients.

20. Use of the engineered cell of any one of claims 6-18 or the pharmaceutical composition of claim 19 in the preparation of a medicament for the prevention and/or treatment of cancer, infection or autoimmune diseases.
